# EUROPEAN PATENT SPECIFICATION

(11) **EP 2 801 347 B1**
(45) Date of publication and mention of the grant of the patent: **07.08.2019**
(21) Application number: 13167254.5
(22) Date of filing: 10.05.2013
(51) Int. Cl.: A61K 8/368, A61K 8/37, A61Q 19/00, A61Q 19/08, A61P 17/00

(54) **Carboxylated stilbenes for activating AMPK and sirtuins**
Carboxylierte Stilbene zur Aktivierung von AMPK und Sirtuinen
Stilbènes carboxylés destinés à activer l'AMPK et des sirtuines

(43) Date of publication of application: 12.11.2014
(73) Proprietor: Rahn Ag, 8050 Zürich (CH)
(72) Inventor: Bitzer, Jens, 44225 Dortmund (DE); Küper, Thomas, 44137 Dortmund (DE); Wabnitz, Philipp, 40237 Düsseldorf (DE)
(74) Representative: Habermann, Hruschka & Schnabel

(56) References cited:
- EP-A2- 2 366 375
- WO-A1-01/74753
- FR-A1- 2 777 186
- JP-A- 2000 007 546
- KAKEGAWA HIASO ET AL: "Inhibitory effects of hydrangenol derivatives on the activation of hyaluronidase and their antiallergic activities", PLANTA MEDICA, THIEME VERLAG, DE, vol. 54, no. 5, 1 January 1988 (1988-01-01), pages 385-389, XP009173666, ISSN: 0032-0943
- ZHANG ET AL: "New type of anti-diabetic compounds from the processed leaves of Hydrangea macrophylla var. thunbergii (Hydrangeae Dulcis Folium)", BIOORGANIC & MEDICINAL CHEMISTRY LETTERS, PERGAMON, GB, vol. 17, no. 17, 4 August 2007 (2007-08-04), pages 4972-4976, XP022184946, ISSN: 0960-894X, DOI: 10.1016/J.BMCL.2007.06.027
- ZHANG H ET AL: "Hydrangeic acid from the processed leaves of Hydrangea macrophylla var. thunbergii as a new type of anti-diabetic compound", EUROPEAN JOURNAL OF PHARMACOLOGY, ELSEVIER SCIENCE, NL, vol. 606, no. 1-3, 15 March 2009 (2009-03-15), pages 255-261, XP026002341, ISSN: 0014-2999, DOI: 10.1016/J.EJPHAR.2009.01.005 [retrieved on 2009-01-17]
- HASHIMOTO T ET AL: "A Highly efficient preparation of lunularic acid and some biological activities of stilbene and dihydrostilbene derivatives", PHYTOCHEMISTRY, PERGAMON PRESS, GB, vol. 27, no. 1, 1 January 1988 (1988-01-01), pages 109-113, XP027094352, ISSN: 0031-9422 [retrieved on 1988-01-01]
- HASHIMOTO T ET AL: "Three dihydroisocoumarin glucosides from Hydrangea macrophylla subsp. serrata", PHYTOCHEMISTRY, PERGAMON PRESS, GB, vol. 26, no. 12, 1 January 1987 (1987-01-01), pages 3323-3330, XP026605741, ISSN: 0031-9422, DOI: 10.1016/S0031-9422(00)82497-8 [retrieved on 1987-01-01]
- NEIL B. RUDERMAN ET AL: "AMPK and SIRT1: a long-standing partnership?", AMERICAN JOURNAL OF PHYSIOLOGY: ENDOCRINOLOGY AND METABOLISM., vol. 298, no. 4, 1 April 2010 (2010-04-01) , pages E751-E760, XP055546145, US ISSN: 0193-1849, DOI: 10.1152/ajpendo.00745.2009
- DUSTIN M. BERMUDEZ ET AL: "Impaired Biomechanical Properties of Diabetic Skin", AMERICAN JOURNAL OF PATHOLOGY., vol. 178, no. 5, 1 May 2011 (2011-05-01), pages 2215-2223, XP055546146, US ISSN: 0002-9440, DOI: 10.1016/j.ajpath.2011.01.015

## Description

The present invention relates to the use of carboxylated stilbene derivatives as defined in claim 1 for cosmetic skin care. The invention is also directed to carboxylated stilbene derivatives as defined in claim 1 for use in the treatment of diabetic skin.

There is an increasing interest in stilbenoids such as trans-reserveratrol for nutraceutical, cosmetic, and putatively pharmaceutical uses. Amongst other effects, transtrans-resveratrol and related hydrostilbenes are sirtuin agonists (US-A-2012/0172340) and activate AMPK (Baur (2010) Biochim. Biophys. Acta 1804(8), 1626-1634).

Hashimoto et al. (1988) Phytochemistry 27, 109-113, disclose dihydrostilbene derivatives obtained by semi-synthetic preparation from *Hydrangea* extracts.

Kakegawa et al. (1988) Planta Medica 53 (5), 385-389, describe anti-allergenic properties of hydrangenol derivatives.

Zhang et al. (2007) Bioorg. Med. Chem. Lett. 17 (17), 4972-4976, disclose anti-diabetic compounds prepared from the leaves of *Hydrangea macrophylla var. thunbergii.*

WO 01/74753 A1 discloses derivatives of lunularic acid and their application as cancer preventive agents.

Zhang et al. (2009) Eur. J. Pharmacol. 606 (1-3), 255-261, describe hydrangeic acid as a new type of anti-diabetic compound.

EP 2 366 375 A2 discloses compositions containing certain quarternary ammonium compounds as sirtuin activators.

JP 2000 007546 A discloses that hydrangenol derivatives are useful as pigmentation preventing agents.

The technical problem underlying the present invention is to provide improved cosmetic and dermatological formulations containing alternative stilbenoid compounds inspired by nature. The solution to the above technical problem is provided by the embodiments of the present invention as described herein and in the claims.

In particular, the present invention provides the use a compound of general formula (I) for cosmetic skin care 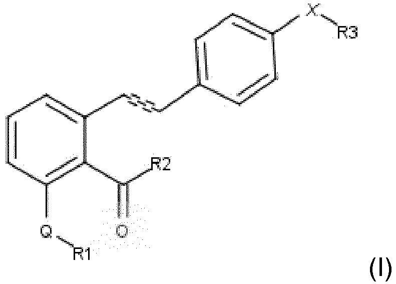 wherein 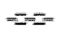 is a single or double bond;
- R¹: is independently hydrogen or a linear, branched or cyclic, saturated or unsaturated C₁₋₆-aliphatic residue, optionally substituted with at least one substituent selected from the group consisting of oxo (=O), -OH, -O-C₁₋₆-alkyl, -C₁₋₆-alkyl, -O-benzyl, phenyl, F, Cl, and Br;
- R²: is independently hydrogen or a linear, branched or cyclic, saturated or unsaturated C₁₋₆-aliphatic residue, optionally substituted with at least one substituent selected from the group consisting of oxo (=O), -OH, -O-C₁₋₆-alkyl, -C₁₋₆-alkyl, -O-benzyl, phenyl, F, Cl, and Br or an aromatic or heteroaromatic 6-membered ring, optionally substituted with at least one substituent selected from the group consisting of -OH, -O-C₁₋₆-alkyl,-C₁₋₆-alkyl, -O-benzyl, phenyl, F, Cl, and Br:
or R² is A-R⁴ wherein
R⁴ is independently hydrogen or a linear, branched or cyclic, saturated or unsaturated C₁₋₆-aliphatic residue, optionally substituted with at least one substituent selected from the group consisting of oxo (=O), -OH, -O-C₁₋₆-alkyl, -C₁₋₆-alkyl, -O-benzyl, phenyl, F, Cl, and Br;
A is O or NR⁵ wherein
R⁵ is independently hydrogen or a linear, branched or cyclic, saturated or unsaturated C₁₋₆-aliphatic residue, optionally substituted with at least one substituent selected from the group consisting of oxo (=O), -OH,-O-C₁₋₆-alkyl, -C₁₋₆-alkyl, -O-benzyl, phenyl, F, Cl, and Br;
- R³: is independently hydrogen or a linear, branched or cyclic, saturated or unsaturated C₁₋₆-aliphatic residue, optionally substituted with at least one substituent selected from the group consisting of oxo (=O), -OH, -O-C₁₋₆-alkyl, -C₁₋₆-alkyl, -O-benzyl, phenyl, F, Cl, and Br, or an aromatic or heteroaromatic 6-membered ring, optionally substituted with at least one substituent selected from the group consisting of -OH, -O-C₁₋₆-alkyl,-C₁₋₆-alkyl, -O-benzyl, phenyl, F, Cl, and Br;
- X: is O or NR⁶ wherein
R⁶ is independently as defined above for R⁵;
- Q: is O or NR⁷ wherein
R⁷ is independently as defined above for R⁵; and
wherein each of the phenyl rings in formula (I) can have one or more further substituents, preferably selected from OH, NH₂, NHR⁸ wherein R⁸ is independently as defined above for R⁵, NR⁹R¹⁰ wherein R⁹,R¹⁰ are independently as defined above for R⁵; alkyl such as C₁₋₆-alkyl, -O-alkyl such as -O-C₁₋₆-alkyl, alkenyl such as C₂₋₆-alkenyl, -O-alkenyl such as -O-C₂₋₆-alkenyl, cycloalkyl such as C₄₋₆-cycloalkyl, heterocycloalkyl such as 4- to 6-membered heterocycloalkyl, aralkyl such as benzyl, -O-aralkyl such as-O-benzyl, aryl such as phenyl, heteroaryl such as 4- to 6-membered heteroaryl, halo such as F, Cl, Br, -CN, -SO₃H, -COOH and corresponding esters such as C₁₋₆-alkyl esters and alkyl amides such as C₁₋₆-alkyl amides, sulfonamides, and O- or-C-glycosidic bound sugars;
optionally, in the form of the corresponding pharmaceutically acceptable salts and/or solvates thereof.

In case 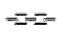 in the above formula (I) is a double bond, the stilbenoid compound is preferably a trans-stilbene, i,e. the double bond is a trans-configured double bond.

In preferred compounds of formula (I) for use in the invention R¹ is hydrogen or C₁₋₃-alkyl. In further preferred embodiments of the invention, R² is hydroxyl or a lower alkoxy group, preferably C₁₋₆-alkoxy, more preferably methoxy or ethoxy. Further preferred compounds of formula (I) for use in the invention are compounds wherein R³ is a lower alkyl group, in particular C₁₋₆-alkyl, more preferably methyl or ethyl. In further preferred compounds of the above formula (I) X is O. In still further preferred compounds of the above formula (I) for use in the invention Q is O.

Particularly preferred compounds for use in the present invention are those in which in formula (I)
- R¹: is hydrogen or C₁₋₃-alkyl;
- R²: is selected from hydroxy, methoxy and ethoxy;
- R³: is C₁₋₆-alkyl, more preferably methyl or ethyl;
- X: is O; and
- Q: is O.

Examples of highly preferred compounds for use in the invention have the following structures:

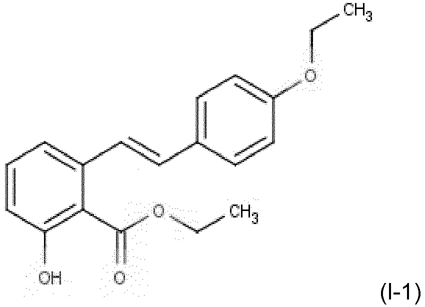

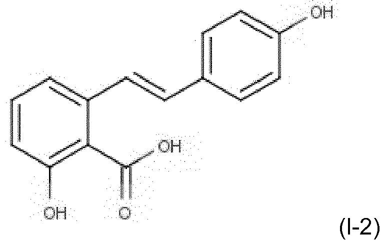

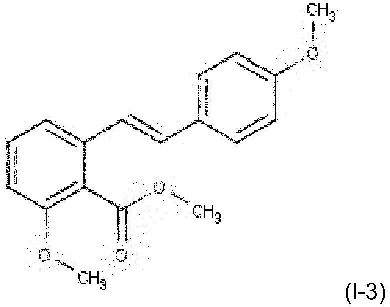

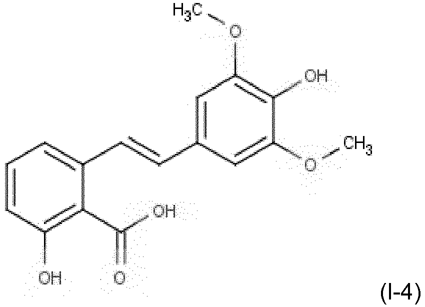

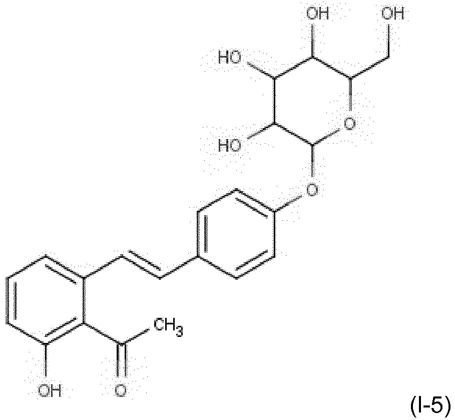

Most preferred, the compound of structure (I-1) is used in the embodiments of the invention.

It is to be understood that all the above compounds include their cosmetically and/or dermatologically acceptable salts and/or solvates, preferably hydrates, of these compounds.

Compounds for use in the present invention having structures as defined above may be prepared by synthesis starting from hydrangenol 8-β-D-glucoside which may be prepared from leaves of *Hydrange macrophylla* as disclosed in Hashimoto et al., *supra,* and according to synthetic principles described therein and general synthesis routes known to the skilled person.

According to the invention, it has been surprisingly found that the compounds of formula (I) are agonists of sirtuins, in particular sirtuins 1, 2 and/or 3, significantly enhance AMPK phosphorylation, and/or significantly increase the number of mitochondria in cells, preferably in skin cells, more preferably human skin cells. Such use according to the invention may, e.g. be *in vitro* or *in vivo.* In particular, the compounds for use in the invention show a greater efficacy, e.g. a higher sirtuin activation, in comparison to trans-resveratrol which is described in the prior art as the most widely used sirtuin activator (see, e.g., Baur (2010), *supra*).

The above characteristics (or at least one of them) make the compounds of formula (I) particularly suitable for cosmetic and medical, i.e. dermatological, applications, preferably as cosmetic and dermatological skin care products, more preferably as anti-aging actives, typically leading, amongst other effects, to skin tightening, reduction of wrinkles and/or complexion improvement.

The invention further relates to cosmetic and dermatological compositions useful in cosmetic and dermatological, respectively, skin care, which compositions contain a compound as defined above together with at least one cosmetically or dermatologically acceptable carrier, excipient and/or diluent.

A "cosmetic composition" or "dermatological composition" as defined herein is a composition comprising a substance according to formula (I) typically in a basic cosmetic or dermatological formulation such that the composition is suitable for application on skin, preferably human skin.

In a preferred embodiment, the composition for use in the present invention comprises a compound of general formula (I), e.g. a compound according to formula (I-1), (I-2), (I-3), (I-4) or (I-5), in an amount of from 0.0001 to 20 wt.-%, preferably 0.001 to 10 wt.-%, more preferably 0.001 to 5 wt.-%, still more preferably 0.001 to 2 wt.-%, yet more preferably 0.01 to 1 wt.-%, most preferably 0.1 to 0.3 wt.-%, based on the total weight of the composition.

In one embodiment, the cosmetic composition for use according to the invention comprises at least one further active ingredient selected from the group consisting of cooling agents, UV radiation absorbers and/or filters, and antioxidants.

In a preferred embodiment, one or more cooling agents are selected from the group consisting of menthone glycerol acetal, also known as Frescolat®MGA, menthyl lactate, also known as Frescolat®ML, wherein menthyl lactate is preferably l-menthyl lactate, more preferably l-menthyl l-lactate, substituted menthyl-3-carboxylic acid amides, e.g. menthyl-3-carboxylic acid N-ethylamide, also known as WS-3, N^{α}-(L-menthanecarbonyl)glycine ethyl ester, also known as WS-5, 2-isopropyl-N-2,3-trimethylbutanamide, also known as WS-23, substituted cyclohexanecarboxylic acid amides, 3-menthoxypropane-1,2-diol, 2-hydroxyethyl menthyl carbonate, 2-hydroxypropyl menthyl carbonate, N-acetylglycine menthyl ester, isopulegol, menthyl hydroxycarboxylic acid esters, e.g. menthyl 3-hydroxybutyrate, monomenthyl succinate, monomenthyl glutarate, 2-mercaptocyclodecanone, menthyl 2-pyrrolidin-5-onecarboxylate, 2,3-dihydroxy-p-menthane, 3,3,5-trimethylcyclohexanone glycerol ketal, 3-menthyl 3,6-di- and -trioxaalkanoates, 3-menthyl methoxyacetate and icilin.

In a preferred embodiment, the total amount of cooling agents in the compositions for use according to the invention is 0.01 to 5 wt.-%, preferably 0.1 to 4 wt.-%, more preferably 0.25 to 3 wt.-%, even more preferably 0.3 to 2.5 wt.-%, in each case based on the total weight of the composition.

Suitable UV filters are e.g. p-aminobenzoic acid, p-aminobenzoic acid ethyl ester (25 mol) ethoxylated, p-dimethylaminobenzoic acid 2-ethylhexyl ester, p-aminobenzoic acid ethyl ester (2 mol) N-propoxylated, p-aminobenzoic acid glycerol ester, salicylic acid homomenthyl ester (homosalate) (Neo Heliopan®HMS), salicylic acid 2-ethylhexyl ester (Neo Heliopan®OS), triethanolamine salicylate, 4-isopropylbenzyl salicylate, anthranilic acid menthyl ester (Neo Heliopan®MA), diisopropylcinnamic acid ethyl ester, p-methoxycinnamic acid 2-ethylhexyl ester (Neo Heliopan®AV), diisopropylcinnamic acid methyl ester, p-methoxycinnamic acid isoamyl ester (Neo Heliopan®E 1000), p-methoxycinnamic acid diethanolamine salt, p-methoxycinnamic acid isopropyl ester, 2-ethylhexyl 2-cyano-3,3-diphenylacrylate (Neo Heliopan®303), ethyl 2-cyano-3,3'-diphenylacrylate, 2-phenylbenzimidazolesulfonic acid and salts (Neo Heliopan®Hydro), 3-(4'-trimethylammonium)-benzylidene-bornan-2-one methyl-sulfate, terephthalylidene- dibornanesulfonic acid and salts (Mexoryl®SX), 4-t-butyl-4'-methoxy-dibenzoylmethane (avobenzone) / (Neo Heliopan®357), [beta]-Imidazole-4(5)-acrylic acid (urocanic acid), 2-hydroxy-4-methoxybenzophenone (Neo Heliopan®BB), 2-hydroxy-4-methoxybenzophenone-5-sulfonic acid, dihydroxy-4-methoxybenzophenone, 2,4-dihydroxybenzophenone, tetrahydroxybenzophenone, 2,2'-dihydroxy-4,4'-dimethoxybenzophenone, 2-hydroxy-4-n-octoxybenzophenone, 2-hydroxy-4-methoxy-4'-methylbenzophenone, 3-(4'-sulfo)benzylidene-bornan-2-one and salts, 3-(4'-methylbenzylidene)-d,l-cmphor (Neo Heliopan®MBC), 3-benzylidene-d,l-camphor, A-isopropyldibenzoylmethane, 2,4,6-trianilino-(p-carbo-2'-ethylhexyl-1'-oxy)-1,3,5-triazine, phenylene-bis-benzimidazyl-tetrasulfonic acid disodium salt (Neo Heliopan®AP), 2,2'-(1,4-phenylene)-bis-(1 H-benzimidazole-4,6-disulfonic acid), monosodium salt, N-[(2 and 4)-[2-(oxoborn-3-ylidene)methyl]benzyl]-acrylamide polymer, phenol, 2-(2H-benzotriazol-2-yl)-4-methyl-6-(2-methyl-3(1,3,3,3-tetramethyl-1-(trimethylsilyl)-oxy)-disiloxyanyl)-propyl), (Mexoryl®XL), 4,4'-[(6-[4-(1,1-dimethyl)-aminocarbonyl)-phenylmino]-1,3,5-triazine-2,4-diyl)diimino]-bis-(benzoic acid 2-ethylhexyl ester) (Uvasorb®HEB), 2,2'-methylene-bis-(6-(2H-benzotriazol-2-yl)-4-(1,1,3,3-tetramethylbutyl)-phenol), (Tinosorb®M), 2,4-bis-[4-(2-ethylhexyloxy)-2-hydroxyphenyl]-1,3,5-triazine, benzylidene malonate-polysiloxane (Parsol®SLX), glyceryl ethylhexanoate dimethoxycinnamate, disodium 2,2'-dihydroxy-4,4'-dimethoxy-5,5'-disulfobenzophenone, dipropylene glycol salicylate, sodium hydroxymethoxybenzophenonesulfonate, 4,4',4-(1,3,5-triazine-2,4,6-triyltriimino)-tris-benzoic acid tris(2-ethylhexyl ester) (Uvinul®T150), 2,4-bis-[{(4-(2-ethyl-hexyloxy)-2-hydroxy}-phenyl]-6-(4-methoxyphenyl)-1,3,5-triazine, (Tinosorb®S), 2,4-bis-[{(4-(3-sulfonato)-2-hydroxy-propyloxy)-2-hydroxy}-phenyl]-6-(4-methoxyphenyl)-1,3,5-triazine sodium salt, 2,4-bis-[{(3-(2-propyloxy)-2-hydroxy-propyloxy)-2-hydroxy}-phenyl]-6-(4-methoxy-phenyl)-1,3,5-triazine, 2,4-bis-[{4-(2-ethyl-hexyloxy)-2-hydroxy}-phenyl]-6-[4-(2-methoxyethyl-carbonyl)-phenylamino]-1 ,3,5-triazine, 2,4-bis-[{4-(3-(2-propyloxy)-2-hydroxy-propyloxy)-2-hydroxy}-phenyl] -6-[4-(2-ethylcarboxyl)-phenylamino]-1 ,3,5- triazine, 2,4-bis-[{4-(2-ethyl-hexyloxy)-2-hydroxy}-phenyl]-6-(1-methyl-pyrrol-2-yl)-1,3,5-triazine, 2,4-bis-[{4-tris-(trimethylsiloxy-silylpropyloxy)-2-hydroxy}-phenyl]-6-(4-methoxyphenyl)-1,3,5-triazine, 2,4-bis-[{4-(2"-methylpropenyloxy)-2-hydroxy}-phenyl]-6-(4-methoxyphenyl)-1 ,3,5-triazine, 2,4-bis-[{4-(1',1',1',3',5',5',5'-heptamethylsiloxy-2"-methyl-propyloxy)-2-hydroxy}-phenyl]-6-(4-methoxyphenyl)-1,3,5-triazine, 2-(4-diethylamino-2-hydroxybenzoyl)-benzoic acid hexyl ester (Uvinul® A Plus) and indanylidene compounds according to DE 100 55 940 (= WO-A-02/38537).

UV absorbers which are particularly suitable for combination are p-aminobenzoic acid, 3-(4'-trimethylammonium)-benzylidene-bornan-2-one methyl-sulfate, salicylic acid homomenthyl ester (Neo Heliopan®HMS), 2-hydroxy-4-methoxy-benzophenone (Neo Heliopan®BB), 2-phenylbenzimidazolesulfonic acid (Neo Heliopan®Hydro), terephthalylidene-dibornanesulfonic acid and salts (Mexoryl®SX), 4-tert-butyl-4'-methoxydibenzoylmethane (Neo Heliopan®357), 3-(4'-sulfo)benzylidene-bornan-2-one and salts, 2-ethylhexyl 2-cyano-3,3-diphenylacrylate (Neo Heliopan®303), N-[(2 and 4)-[2-(oxoborn-3-ylidene)methyl]benzyl]-acrylmide polymer, p-methoxycinnamic acid 2-ethylhexyl ester (Neo Heliopan®AV), p-aminobenzoic acid ethyl ester (25 mol) ethoxylated, p-methoxycinnamic acid isoamyl ester (Neo Heliopan®E1000), 2,4,6-trianilino-(p-carbo-2'-ethylhexyl-1'-oxy)-1,3,5-triazine (Uvinul®T150), phenol, 2-(2H-benzotriazol-2-yl)-4-methyl-6-(2-methyl-3(1,3,3,3- tetramethyl-i-(trimethylsilyl)-oxy)-disiloxyanyl)-propyl), (Mexoryl®XL), 4,4'-[(6-[4-(1,1-dimethyl)-aminocarbonyl)-phenylamino]-1,3,5-triazine-2,4-diyl)-diimino]-bis-(benzoic acid 2-ethylhexyl ester), (UvasorbHEB), 3-(4'-methylbenzylidene)-d,l-cmphor (Neo Heliopan®MBC), 3-benzylidenecamphor, salicylic acid 2-ethylhexyl ester (Neo Heliopan®OS), 4-dimethylaminobenzoic acid 2-ethylhexyl ester (Padimate O), hydroxy-4-methoxybenzophenone-5-sulfonic acid and Na salt, 2,2'-methylene-bis-(6-(2H-benzotriazol-2-yl)-4-(1,1,3,3-tetramethylbutyl)-phenol), (Tinosorb®M), phenylene-bis-benzimidazyl-tetrasulfonic acid disodium salt (Neo Heliopan®AP), 2,4-bis-[{(4-(2-ethyl-hexyloxy)-2-hydroxy}-phenyl]-6-(4-methoxyphenyl)-1,3,5-triazine, (Tinosorb®S), benzylidene malonate-polysiloxane (Parsol®SLX), menthyl anthranilate (Neo Heliopan®MA), 2-(4-diethylamino-2-hydroxybenzoyl)-benzoic acid hexyl ester (Uvinul® A Plus) and indanylidene compounds according to DE 100 55 940 (= WO-A-02/38537).

Suitable UV absorbers and/or filters are also sunscreen pigments such as finely dispersed metal oxides and metal salts, for example titanium dioxides, zinc oxide (ZnO), iron oxides (e.g. Fe₂O₃), aluminium oxide (Al₂O₃); cerium oxides (e.g. Ce₂O₃), manganese oxides (e.g. MnO), zirconium oxide (ZrO₂), silicon oxide (SiO₂), mixed oxides of the corresponding metals and mixtures of such oxides, barium sulfate and zinc stearate. They are particularly preferably pigments based on TiO₂ or zinc oxide. In preferred embodiments, the particles have an average diameter of less than 100 nm, preferably between 5 and 50 nm and particularly preferably between 15 and 30 nm. They can have a spherical shape, but those particles which have an ellipsoid shape or a shape which deviates otherwise from the spherical can also be employed. The pigments can also be in a form treated on the surface, i.e. hydrophilized or hydrophobized. Typical examples are coated titanium dioxides, such as e.g. titanium dioxide T 805 (Degussa) or Eusolex® T2000 (Merck), or coated zinc oxide, such as e.g. Zinc Oxide NDM. In this context, possible hydrophobic coating agents are, above all, silicones, and in this case specifically trialkoxyoctysilanes or simethicone. So-called micro- or nanopigments are preferably employed in sunscreen compositions. Zinc micro- or nanopigments are preferably employed.

In a preferred embodiment, the total amount of UV radiation absorbers and/or filters in the compositions for use according to the invention is 0.01 to 20 wt.-%, preferably 0.05 to 10 wt.-%, more preferably 0.2 to 5 wt.-%, based on the total weight of the composition.

According to preferred embodiments, one or more antioxidants are selected from the group consisting of amino acids, preferably glycine, histidine, tyrosine, tryptophan, and derivatives thereof, imidazoles, preferably urocanic acid, and derivatives thereof, peptides, preferably D,L-carnosine, D-carnosine, L-carnosine and derivatives thereof, preferably anserine, carotenoids, carotenes, preferably α-carotene, β-carotene, lycopene, and derivatives thereof, chlorogenic acid and derivatives thereof, liponic acid and derivatives thereof, preferably dihydroliponic acid, aurothioglucose, propylthiouracil and other thiols, preferably thioredoxin, glutathione, cysteine, cystine, cystamine and glycosyl, N-acetyl, methyl, ethyl, propyl, amyl, butyl and lauryl, palmitoyl, oleyl, γ-linoleyl, cholesteryl and glyceryl esters thereof, and salts thereof, dilauryl thiodipropionate, distearyl thiodipropionate, thiodipropionic acid and esters, ethers, peptides, lipids, nucleotides, nucleosides and salts thereof, (metal) chelators, preferably α-hydroxy-fatty acids, palmitic acid, phytic acid, lactoferrin, α-hydroxy acids, preferably citric acid, lactic acid, malic acid, humic acid, bile acid, bile extracts, bilirubin, biliverdin, EDTA, EGTA and derivatives thereof, unsaturated fatty acids and derivatives thereof, preferably γ-linolenic acid, linoleic acid, oleic acid, folic acid and derivatives thereof, ubiquinone and ubiquinol and derivatives thereof, vitamin C and derivatives, preferably ascorbyl palmitate, Mg ascorbyl phosphate, ascorbyl acetate, ascorbyl glycosides, preferably 6-O-acyl-2-O-α-D-glucopyranosyl-L-ascorbic acid, 6-O-acyl-2-O-β-D-glucopyranosyl-L-ascorbic acid, 2-O-α-D-glucopyranosyl-L-ascorbic acid or 2-O-β-D-glucopyranosyl-L-ascorbic acid, tocopherols and derivatives thereof, preferably vitamin E acetate, vitamin A and derivatives thereof, preferably vitamin A palmitate as well as coniferylbenzoate of benzoin resin, rutic acid and derivatives thereof, α-glucosylrutin, quercetin and derivatives thereof, rosemary acid, carnosol, carnosol acid, resveratrol, caffeic acid and derivatives thereof, sinapic acid and derivatives thereof, ferulic acid and derivatives thereof, furfurylideneglucitol, curcuminoids, butylhydroxytoluene, butylhydroxyanisole, nordihydroguaiac resin acid, nordihydroguaiaretic acid, trihydroxybutyrophenone, uric acid and derivatives thereof, mannose and derivatives thereof, superoxide dismutase, zinc and derivatives thereof, preferably ZnO, ZnSO₄, selenium and derivatives thereof, preferably selenium methionine, stilbenes and derivatives thereof, preferably stilbene oxide, trans-stilbene oxide and derivatives, preferably salts, esters, ethers, sugars, nucleotides, nucleosides, peptides and lipids, of these active compounds mentioned and combinations thereof. Alternatively or additionally antioxidatively active extracts or fractions from plants selected from the group consisting of green tea, rooibos, honeybush, grape, rosemary, sage, Melissa, thyme, lavender, olive, oats, cocoa, ginkgo, ginseng, liquorice, honeysuckle, Sophora, Pueraria, Pinus, Citrus, Phyllanthus emblica, St. John's wort and combinations thereof are suitable antioxidants.

In a preferred embodiment, the total amount of antioxidants in the compositions for use according to the invention is preferably 0.01 to 20 wt.-%, particularly preferably 0.05 to 10 wt.-%, in particular 0.2 to 5 wt.-%, based on the total weight of the composition.

In further embodiments, the compositions for use as defined herein contain a preservative as a further ingredient, which may preferably be selected from the group consisting of benzoic acid and its esters and salts, 4-hydroxybenzoic acid and its esters (INCI: Parabens, preferably methylparaben, ethylparaben, butylparaben, propylparaben and/or isobutylparaben) and salts, propionic acid and its esters and salts, salicylic acid and its esters and salts, 2,4-hexadienoic acid (sorbic acid) and its esters and salts, formaldehyde and paraformaldehyde, 2-hydroxybiphenyl ether and its salts, 2-zinc-sulfidopyridine N-oxide, inorganic sulfites and bisulfites, sodium iodate, chlorobutanolum, 4-ethylmercury(II)-5-amino-1,3-bis(2-hydroxybenzoic acid), its salts and esters, dehydracetic acid, formic acid, 1,6-bis(4-amidino-2-bromophenoxy)-n-hexane and its salts, the sodium salt of ethylmercury-(II)-thiosalicylic acid, phenylmercury and its salts, 10-undecylenic acid and its salts, 5-amino-1,3-bis(2-ethylhexyl)-5-methyl-hexahydropyrimidine, 5-bromo-5-nitro-1,3-dioxane, 2-bromo-2-nitro-1,3-propanediol, 2,4-dichlorobenzyl alcohol, N-(4-chlorophenyl)-N'-(3,4-dichlorophenyl)-urea, 4-chloro-m-cresol, 2,4,4'-trichloro-2'-hydroxy-diphenyl ether, 4-chloro-3,5-dimethylphenol, 1,1'-methylene-bis(3-(1-hydroxymethyl-2,4-dioximidazolidin-5-yl)urea), poly-(hexamethylenediguanide) hydrochloride, 2-phenoxyethanol, hexamethylenetetramine, 1-(3-chloroallyl)-3,5,7-triaza-1-azonia- adamantane chloride, 1-(4-chlorophenoxy)-1-(1 H-imidazol-1-yl)-3,3-dimethyl-2-butanone, 1,3-bis-(hydroxy-methyl)-5,5-dimethyl-2,4-imidazolidinedione, benzyl alcohol, Octopirox, 1,2-dibromo-2,4-dicyanobutane, 2,2'-methylene-bis(6-bromo-4-chlorophenol), bromochlorophene, mixture of 5-chloro-2-methyl-3(2H)-isothiazolinone and 2-methyl-3(2H)-isothiazolinone with magnesium chloride and magnesium nitrate, 2-benzyl-4-chlorophenol, 2-chloroacetamide, chlorhexidine, chlorhexidine acetate, chlorhexidine gluconate, chlorhexidine hydrochloride, 1-phenoxy-propan-2-ol, N-alkyl(C₁₂-C₂₂)trimethylammonium bromide and chloride, 4,4-dimethyl-1,3-oxazolidine, N-hydroxymethyl-N-(1,3-di(hydroxymethyl)-2,5-dioxoimidazolidin-4-yl)-N'-hydroxy-methylurea, 1,6-bis(4-amidino-phenoxy)-n-hexane and its salts, glutaraldehyde, 5-ethyl-1-aza-3,7-dioxabicyclo(3.3.0)octane, 3-(4-chlorophenoxy)-1,2-propanediol, hyamines, alkyl-(C₈-C₁₈)-dimethyl-benzyl-ammonium chloride, alkyl-(C₈-C₁₈)-dimethyl-benzylammonium bromide, alkyl-(C₈-C₁₈)-dimethyl-benzylammonium saccharinate, benzyl hemiformal, 3-iodo-2-propynyl butylcarbamate, sodium hydroxymethyl-aminoacetate or sodium hydroxymethyl-aminoacetate.

Cosmetically acceptable carriers applicable in compositions for use in the invention are preferably selected from the group consisting of
(I) one or more diols, preferably alkane diol(s), having 3 to 10 carbon atoms, preferably selected from the group consisting of 1,2-propylene glycol, 2-methylpropane-1,3-diol, 1,2-butylene glycol, 1,3-butanediol, 1,2-pentanediol, 1,3-pentanediol, 1,5-pentanediol, 2,4-pentanediol, 2-methyl-pentane-2,4-diol, 1,2-hexanediol, 1,6-hexanediol, 1,2-octanediol, dipropylene glycol, preferably 1,2-butylene glycol, 1,2-pentanediol and/or dipropylene glycol, and/or
(II) a cosmetically acceptable carrier having a Clog P value of at least 4, preferably of at least 5, more preferably of at least 6, and preferably selected from groups (II-1) and/or (II-2) and/or (II-3) or mixtures thereof, said groups consisting of
   (II-1) esters having 6 to 36 carbon atoms, preferably monoesters, diesters or triesters, preferably selected from the group consisting of diethyl phthalate, diethylhexyl 2,6-naphthalate, isopropyl myristate, isopropyl palmitate, isopropyl stearate, isopropyl oleate, n-butyl stearate, n-hexyl laurate, n-decyl oleate, isooctyl stearate, isononyl stearate, isononyl isononanoate, 3,5,5-trimethylhexyl 3,5,5-trimethylhexanoate, 2-ethylhexyl isononanoate, 2-ethylhexyl 3,5,5-trimethylhexanoate, 2-ethylhexyl 2-ethylhexanoate, 2-ethylhexyl palmitate, 2-ethylhexyl laurate, 2-hexyldecyl stearate, cetearyl ethylhexanoate, stearyl heptanoate, stearyl caprylate, 2-octyldodecyl palmitate, oleyl oleate, oleyl erucate, erucyl oleate, erucyl erucate, 2-ethylhexyl isostearate, isotridecyl isononanoate, 2-ethylhexyl cocoate, C₁₂₋₁₅-alkyl benzoates, cetyl palmitate, triethyl citrate, triacetin (triacetyl citrate), benzyl benzoate, benzyl acetate, vegetable oils (preferably olive oil, sunflower oil, soya oil, groundnut oil, rapeseed oil, almond oil, palm oil, coconut oil, palm kernel oil) and triglycerides, in particular glyceryl stearate, glyceryl triisononanoate, glyceryl laurate or triglycerides with identical or different C6 to C10 fatty acid radicals (so-called medium-chain triglycerides, in particular caprylic/capric triglyceride, like glyceryl tricaprylate, glyceryl tricaprate), and/or
   (II-2) branched and unbranched alkyl or alkenyl alkohols, preferably selected from the group consisting of decanol, decenol, octanol, octenol, dodecanol, dodecenol, octadienol, decadienol, dodecadienol, oleyl alcohol, ricinoleyl alcohol, erucyl alcohol, stearyl alcohol, isostearyl alcohol, cetyl alcohol, lauryl alcohol, myristyl alcohol, arachidyl alcohol, linoleyl alcohol, linolenyl alcohol, hexyldecanol, octyldodecanol (in particular 2-octyl-1 -dodecanol) and cetearyl alcohol and behenyl alcohol, and/or
   (II-3) branched and unbranched hydrocarbons and waxes, cyclic or linear silicone oils and dialkyl ethers having 6 to 24 carbon atoms, preferably selected from the group consisting of jojoba oil, isoeicosane, dicaprylyl ether, mineral oil, petrolatum, squalane, squalene, cyclomethicone, decamethylcyclopentasiloxane, undecamethylcyclotrisiloxane, polydimethylsiloxane and poly(methyl-phenyl siloxane.

The Clog P value (also known as log Pow) is the decimal logarithm of the distribution coefficient of a substance or material between 1-octanol and water. Clog P values are well known in the chemical arts as a calculated value that represents the relative affinity that a substance or material has for partitioning between octanol and water. Clog P values can be obtained or calculated as described and referenced in WO-A-2008/114189.

In a preferred embodiment, the compositions for use according to the invention comprise a total amount of 5 to 70 wt.-%, preferably 7.5 to 60 wt.-%, more preferably 10 to 50 wt.-%, even more preferably 10 to 40 wt.-%, of one or more cosmetically acceptable carriers as defined above, in each case based on the total weight of the composition.

In one embodiment, the compositions for use according to the invention comprise water in an amount of up to 98 wt.-%, preferably 10 to 95 wt.-%, more preferably 25 to 90 wt.-%, in each case based on the total weight of the composition.

In one embodiment, the compositions for use according to the invention comprise at least one further ingredient selected from the group consisting of moisture retention regulators, anti-inflammatory agents and skin care agents, anti-aging agents, vitamins and vitamin precursors, plant extracts and fragrances.

The present invention further relates to a cosmetic method for skin care, preferably for the prevention and/or treatment of skin aging, comprising the step of topically applying an effective amount of a cosmetic composition as defined above onto human skin.

The present invention also relates to the dermatological use of the compound as defined above for the treatment of diabetic skin. For such use according to the invention an effective amount of a dermatological composition as defined above is typically applied onto skin of a human in need of such treatment.

An effective amount of a composition as defined herein is typically 0.1 to 5 g of the compositions applied to about 10 cm² of human skin.

The compositions of the invention may be applied to human skin at least once in a week, preferably once a day, more preferably twice a day.

The composition of the invention for cosmetic or dermatological use may take the form of or may be contained in, respectively, a typical cosmetic or dermatological product being preferably selected from the group consisting of soap, synthetic detergent, liquid washing, shower and bath preparation, skin care product, perfume, sunscreen product, after-sun product, foot care product, depilatory product, hair care product, deodorant, antiperspirant, mouthwash, ophthalmic preparation, nasal care product, makeup, makeup remover, decorative cosmetics, shaving product and after-shave product.

Exemplary skin care products include day and night creams, body lotions and body sprays. The day creams, skin lotions and night creams preferably include O/W-day creams, O/W-skin lotions and O/W-night creams, respectively.

Perfumes preferably also include eau de toilettes and eau de colognes.

Sunscreen products preferably include balms, lotions and milks. After-sun products preferably include balms, lotions and milks.

Foot care products preferably include keratolytics and deodorants.

Hair care products preferably include shampoos, preferably 2-in-1-shampoos, conditioners, hair tonic waters, hair rinses, hair creams, pomade, perm and setting lotions, hair smoothing products, preferably detangling products or relaxers, hair strengtheners, styling aids, preferably gels or waxes, blonding products, hair dyes, preferably temporary hair dyes, colour rinses, semi-permanent and permanent hair-dyes.

Decorative cosmetics preferably include powders, eyeshadows, kohl pencils and lipsticks.

Exemplary shaving products preferably include shaving foam, shaving gel, shaving soap and shaving lather. Exemplary after-shave products preferably include after-shave balm, after-shave lotion, after-shave splash, and after-shave.

In one embodiment, the cosmetic or dermatological product for use according to the invention is in the form of a solution, dispersion, suspension, cream, lotion, milk, emulsion, ointment, suppository, paste, gel, oil, toner, balsam, serum, powder, wax, stick, spray, aerosol, or wipe.

Emulsions preferably include W/O-, O/W- or multiple emulsions, PIT emulsions, emulsion foams, micro-emulsions, nano-emulsions, and Pickering emulsions. Gels preferably include hydrogels, hydrodispersion gels and oleogels. Aerosols preferably include foaming, non-foaming or post-foaming aerosols. Wipes preferably also include wet wipes.

In a preferred embodiment, the cosmetic or dermatological product for use in the invention is in the form of an emulsion or a wipe, preferably a wet wipe. Most preferably, the cosmetic product according to the invention is in the form of an emulsion.

If the cosmetic or dermatological product to be used according to the invention is in the form of an emulsion, the oily phase is preferably selected from the group consisting of:
- mineral oils, mineral waxes;
- fatty oils, fats, waxes and other natural and synthetic fat substances, preferably esters of fatty acids with alcohols of low C number, e.g. with isopropanol, propylene glycol or glycerol, or esters of fatty alcohols with alkanoic acids of low C number or with fatty acids;
- alkyl benzoates;
- silicone oils, such as dimethylpolysiloxanes, diethylpolysiloxanes, diphenylpolysiloxanes and mixed forms thereof.

Compounds which can advantageously be employed are (a) esters of saturated and/or unsaturated branched and/or linear alkanecarboxylic acids having a chain length of from 3 to 30 C atoms and saturated and/or unsaturated, branched and/or linear alcohols having a chain length of from 3 to 30 C atoms, (b) esters of aromatic carboxylic acids and saturated and/or unsaturated, branched and/or linear alcohols having a chain length of from 3 to 30 C atoms. Preferred ester oils are isopropyl myristate, isopropyl palmitate, isopropyl stearate, isopropyl oleate, n-butyl stearate, n-hexyl laurate, n-decyl oleate, isooctyl stearate, isononyl stearate, isononyl isononanoate, 3,5,5- trimethylhexyl 3,5,5-trimethylhexanoate, 2-ethylhexyl isononanoate, 2-ethylhexyl 3,5,5- trimethylhexanoate, 2-ethylhexyl 2-ethylhexanoate, 2-ethylhexyl palmitate, 2-ethylhexyl laurate, 2-hexyldecyl stearate, 2-octyldodecyl palmitate, oleyl oleate, oleyl erucate, erucyl oleate, erucyl erucate and synthetic, semi-synthetic and natural mixtures of such esters, e.g. jojoba oil.

The oily phase can furthermore advantageously be selected from the group consisting of branched and linear hydrocarbons and waxes, silicone oils and dialkyl ethers, the group consisting of saturated or unsaturated, branched or linear alcohols, and the fatty acid triglycerides, namely the triglycerol esters of saturated and/or unsaturated, branched and/or linear alkanecarboxylic acids having a chain length of from 8 to 24, in particular 12 to 18 C atoms. The fatty acid triglycerides can advantageously be selected from the group consisting of synthetic, semi-synthetic and natural oils, e.g. olive oil, sunflower oil, soya oil, groundnut oil, rapeseed oil, almond oil, palm oil, coconut oil, palm kernel oil and more of the like. Any desired blends of such oil and wax components can also advantageously be employed. In some cases it is also advantageous to employ waxes, for example cetyl palmitate, as the sole lipid component of the oily phase, and the oily phase is advantageously chosen from the group which consists of 2-ethylhexyl isostearate, octyldodecanol, isotridecyl isononanoate, isoeicosane, 2-ethylhexyl cocoate, C₁₂₋₁₅-alkyl benzoate, caprylic/capric acid triglyceride and dicaprylyl ether. Mixtures of C₁₂₋₁₅-alkyl benzoate and 2-ethylhexyl isostearate, mixtures of C₁₂₋₁₅-alkyl benzoate and isotridecyl isononanoate and mixtures of C₁₂₋₁₅-alkyl benzoate, 2-ethylhexyl isostearate and isotridecyl isononanoate are particularly advantageous. The hydrocarbons paraffin oil, squalane and squalene can also advantageously be used. The oily phase can furthermore have a content of cyclic or linear silicone oils or consist entirely of such oils, it being advantageous to use an additional content of other oily phase components in addition to the silicone oil or silicone oils. Cyclomethicone (e.g. decamethylcyclopentasiloxane) can advantageously be employed as a silicone oil. However, other silicone oils, for example undecamethylcyclotrisiloxane, polydimethylsiloxane and poly(methyl-phenylsiloxane), can also advantageously be used. Mixtures of cyclomethicone and isotridecyl isononanoate and of cyclomethicone and 2- ethylhexyl isostearate are furthermore particularly advantageous.

Cosmetic or dermatological products in the form of an emulsion which comprise a composition for use according to the invention advantageously further comprise one or more emulsifiers. O/W emulsifiers can preferably be selected from the group consisting of polyethoxylated or polypropoxylated or polyethoxylated and polypropoxylated products. In a preferred embodiment, the polyethoxylated or polypropoxylated or polyethoxylated and polypropoxylated O/W emulsifiers employed are selected from the group consisting of substances having HLB values of 11 - 18, preferably having HLB values of 14.5 - 15.5, if the O/W emulsifiers contain saturated radicals R and R'. If the O/W emulsifiers contain unsaturated radicals R and/or R', or isoalkyl derivatives are present, the preferred HLB value of such emulsifiers can also be lower or higher. It is particularly preferable to select the fatty alcohol ethoxylates from the group consisting of ethoxylated stearyl alcohols, cetyl alcohols and cetyl stearyl alcohols (cetearyl alcohols). Exemplary W/O emulsifiers which can be employed are: fatty alcohols having 8 to 30 carbon atoms, monoglycerol esters of saturated and/or unsaturated, branched and/or unbranched alkanecarboxylic acids having a chain length of from 8 to 24, in particular 12 to 18 C atoms, diglycerol esters of saturated and/or unsaturated, branched and/or unbranched alkanecarboxylic acids having a chain length of from 8 to 24, in particular 12 to 18 C atoms, monoglycerol ethers of saturated and/or unsaturated, branched and/or unbranched alcohols having a chain length of from 8 to 24, in particular 12 to 18 C atoms, diglycerol ethers of saturated and/or unsaturated, branched and/or unbranched alcohols having a chain length of from 8 to 24, in particular 12 to 18 C atoms, propylene glycol esters of saturated and/or unsaturated, branched and/or unbranched alkanecarboxylic acids having a chain length of from 8 to 24, in particular 12 to 18 C atoms and sorbitan esters of saturated and/or unsaturated, branched and/or unbranched alkanecarboxylic acids having a chain length of from 8 to 24, in particular 12 to 18 C atoms.

The following non-limiting examples further illustrate the present invention.

### EXAMPLES

### Example 1: Agonistic effect on sirtuins

The agonistic effect of compounds of formula (I) on human sirtuin-1 (Sirt1) was determined *in vitro* and compared with the known agonistic effect of trans-resveratrol as benchmark.

Compounds were tested for Sirt1 activation using the SIRT1 Fluorimetric Drug Discovery Kit (BioMol, Hamburg, Germany), Testing was carried out in dubplicate following the manufacturer's instructions. Assay medium: 50mM Tris-HCl pH 8.0, 137 mM NaCl, 2.7 mM KCl, 1 mM MgCl₂, 1 mg/ml bovine serum albumin (BSA). Stock solutions of the test compounds (10 mM in DMSO) were used, yielding a final concentration of 10 µM in the assay plate. Readout was obtained by measuring the fluorescence in a microtiter plate reader (excitation: 380 nm; detection: 440 nm).

### (a) Screening at 10 µM concentration: results are shown in Table 1

**Table 1: Agonistic effect on Sirt1 in comparison to trans-resveratrol**

| **Compound** | **Fold increase in sirt1 activity versus control** |
|---|---|
| (I-1) | 23.9 |
| (I-2) | 4.8 |
| (I-3) | 1.1 |
| (I-4) | 2.0 |
| (I-5) | 2.7 |
| trans-resveratrol (control) | 1.0 |

### (b) Quantification of the Sirt1 agonistic effect, dose response

The agonistic effect on Sirt1 was determined at different concentrations. For this purpose, the test item stock solutions were diluted to yield concentrations of 0.3, 1, 3, 10 and 30 µM, respectively, in the assay, which was performed as described above. The compound of structure (I-1) was selected for this experiment. The dose-response behaviour observed was found clearly superior in comparison to trans-resveratrol (Table 2).

**Table 2: Dose-response effect of compound (I-1) on Sirt1 in comparison to trans-resveratrol**

| **Concentration [µM]** | **Activity [% control]** | |
|---|---|---|
| | compound (I-1) | trans-resveratrol |
| 30.0 | 1381 | 120 |
| 10.0 | 626 | 108 |
| 3.0 | 434 | 100 |
| 1.0 | 233 | 101 |
| 0.3 | 147 | 104 |

Quantification using the GraphPad Prism 5.0 software (GraphPad Software, Inc., LaJolla, CA, USA) led to the following EC300 values (i.e. the concentration that gave 3-times the basal response of the enzyme = activation by a factor of three):
Compound of formula (I-1): EC300 = 2.0 µM
Trans-resveratrol: EC300 > 30 µM

### Example 2: Selectivity of agonistic effect on sirtuins

The selectivity of the agonistic effect of the compound of formula (I-1) on different sirtuin isoforms was investigated. For this purpose, *in vitro* testing was performed as described in Example 1 using the respective SIRT1 Fluorimetric Drug Discovery Kit, SIRT2 Fluorimetric Drug Discovery Kit, and SIRT3 Fluorimetric Drug Discovery Kit (each from BioMol, Hamburg, Germany). The results demonstrate that compound (I-1) has an agonistic acitivity on all three sirtuin isoform and shows similar dose-response behaviours on Sirt1 and Sirt2. The dose-response effect is slightly lesser on Sirt3.

**Table 3: Activity on Sirt1**

| **Concentration [µM]** | **Activity [% control]** | |
|---|---|---|
| | compound (I-1) | trans-resveratrol |
| 30.0 | 515 | 145 |
| 10.0 | 256 | 111 |
| 3.0 | 168 | 117 |
| 1.0 | 121 | 100 |
| 0.3 | 111 | 121 |

**Table 4: Activity on Sirt2**

| **Concentration [µM]** | **Activity [% control]** | |
|---|---|---|
| | compound (I-1) | trans-resveratrol |
| 30.0 | 914 | 105 |
| 10.0 | 530 | 100 |
| 3.0 | 204 | 97 |
| 1.0 | 142 | 104 |
| 0.3 | 114 | 104 |

**Table 5: Activity on Sirt3**

| **Concentration [µM]** | **Activity [% control]** | |
|---|---|---|
| | compound (I-1) | trans-resveratrol |
| 30.0 | 247 | 132 |
| 10.0 | 216 | 113 |
| 3.0 | 156 | 116 |
| 1.0 | 124 | 114 |
| 0.3 | 109 | 105 |

Quantification using the GraphPad Prism 5.0 software (GraphPad Software, Inc., LaJolla, CA, USA) leads to the following EC300 values (Table 6):

**Table 6: EC300 values of compound (I-1) with respect to sirtuin isoforms**

| **Sirtuin isoform** | **EC300 compound (I-1) [µM]** | **EC300 trans-resveratrol [µM]** |
|---|---|---|
| Sirt1 | 12.9 | >30 |
| Sirt2 | 4.9 | >30 |
| Sirt3 | >30* | >30 |

| | | |
|---|---|---|
| * However: clear activation in dose-response curve visible. | | |

### Example 3: Effect of compound (I-1) on AMPK

The effect of compound (I-1) on 5'-AMP-activated protein kinase (AMPK) was measured in normal human epidermal keratinocytes (NHEK). AMPK is activated by phosphorylation as phospho-AMPK (P-AMPK). The degree of phosphorylation was evaluated by Western blot. Glycerinaldehyde-3-phosphate dehydrogenase (GAPDH) was used as housekeeping protein and loading control. The level of AMPK phosphorylation was determined by calculating the P-AMPK/GAPDH ratio. 5-Amino-1-β-D-ribofuranosyl-imidazole-4-carboxamide (AICAR), a known stimulator of AMPK activation (Corton et al. (1995), Eur. J. Biochem. 229, pp. 558-565), was used as positive control.

Culture conditions: 37 °C, 5 % CO₂. Culture medium: Keratinocyte-SFM (serum-free medium) containing human recombinant epidermal growth factor (EGF 1-53) 0.25 ng/mL and bovine pituitary extract 25 µg/mL (Gibco; Cat. No. 17005-075), supplemented with gentamycin 25 µg/mL. Assay medium: Keratinocyte-SFM (Gibco; Cat. No. 10744-019), supplemented with gentamycin 25 µg/mL.

NHEK were seeded in 24-well plates (30.000 cells / well) and cultured in culture medium for 24 h and then in assay medium for another 24 h. Medium was then removed and replaced by assay medium containing the respective test compound or reference in defined concentrations.

Cytotoxicity was determined using the 3-(4,5-dimethylthiazol-2-yl)-2,5-diphenyltetrazoliumbromid (MTT) reduction assay (sse T. Mosmann (1983) J. Immunol. Methods. 65, pp. 55-63). All cells were found to be fully viable except for those incubated with trans-resveratrol at 100 µM (2 % viability) and at 300 µM (43 % viability), illustrating the cytotoxic effect of trans-resveratrol on NHEK at the respective concentrations. At the same concentrations, NHEK cells with compound (I-1) retained full viability.

### Western blot analysis of AMPK phosphorylation

The cells were incubated for 48 h in assay medium containing the test items. Cell lysis was performed using Cell Extraction Buffer (Invitrogen, Cat. No. FNN0011), supplemented with the Phosphatase Inhibitor Cocktail 1 (Sigma-Aldrich, Cat. No. P2850). The protein amount was quantified using the DC™ protein assay (Biorad, Cat. No. 500-0112). Volumes were adjusted in order to load for each condition 20 µg of total proteins per lane. Proteins were denaturized in Laemmli buffer (Biorad, Cat. No. 161-0737) at 95 °C, subjected to electrophoresis on a 10 % polyacrylamide gel, and then transferred onto nitrocellulose membrane. After saturation in phosphate buffered saline (PBS)/Tween/bovine serum albumin (BSA), P-AMPK and GAPDH were successively detected using specific primary antibodies (Anti-P-AMPK, ref #4188, Cell signalling technology; Anti-GAPDH[HRP], ref #ab9482, ABCAM). P-AMPK primary antibody was detected using an appropriate horseradish peroxidise (HRP)-conjugated secondary antibody whereas GAPDH was directly conjugated to HRP. A single blot was developed successively for each of the markers. After each antibody incubation the blot was washed using stripping buffer allowing for fixation of the next antibody. The different markers were developed by enhanced chemiluminescence (ECL Plus Amersham™, GE Healthcare). Image acquisition was performed with a scanner of chemiluminescence (FUJI LAS 3000, Fujifilm); and densitometric analysis was obtained using Multigauge software (Fujifilm).

**Table 7: Experiment 1**

| | Conc. [µM] | P-AMPK [AU/mm²] | GAPDH [AU/mm²] | Ratio P-AMPK/GAPDH | AMPK phosphorylation [% Control] |
|---|---|---|---|---|---|
| *Membrane 1* | | | | | |
| Control | -- | 196 | 463 | 0.42 | 100 |
| AICAR | 1000 | 1060 | 518 | 2.05 | 482 |
| Compound (I-1) | 3 | 123 | 480 | 0.26 | 60 |
| | 10 | 202 | 528 | 0.38 | 90 |
| | 30 | 2554 | 599 | 4.26 | 1006 |

| *Membrane 2* | | | | | |
|---|---|---|---|---|---|
| Control | -- | 296 | 373 | 0.79 | 100 |
| trans-resveratrol | 300 | 1616 | 282 | 5.73 | 722 |

| | | | | | |
|---|---|---|---|---|---|
| AU = arbitrary units | | | | | |

**Table 8: Experiment 2**

| | Conc. [µM] | P-AMPK [AU/mm²] | GAPDH [AU/mm²] | Ratio P-AMPK/GAPDH | AMPK phosphorylation [% Control] |
|---|---|---|---|---|---|
| Control | -- | 249656 | 2208428 | 0.113 | 100 |
| DMSO | 2% | 306555 | 1978162 | 0.155 | 137 |
| AICAR | 1000 | 1377449 | 2406346 | 0.572 | 506 |
| trans-resveratrol | 10 | 228626 | 2511753 | 0.091 | 81 |
| | 30 | 211794 | 2177595 | 0.097 | 86 |
| | 100 | 327757 | 2479461 | 0.132 | 117 |
| | 200 | 2361575 | 2561347 | 0.922 | 816 |
| | 300 | 69290 | 2912376 | 0.024 | 21 |

| | | | | | |
|---|---|---|---|---|---|
| AU = arbitrary units | | | | | |

### Conclusions:

i) Compound (I-1) significantly enhanced AMPK phosphorylation at 30 µM. Surprisingly, trans-resveratrol needed about 10 times higher dosage (at least 200 µM) for a similar effect. Such concentrations (100 µM trans-resveratrol and higher) already had cytotoxic effects on NHEK under assay conditions.
ii) The effect of compound (I-1) is significantly higher than that of the reference AICAR.
iii) Phosphorylation of AMPK in NHEK in presence of trans-resveratrol is observed for such concentrations only which showed cytotoxic effects, whereas no cytotoxicity against NHEK was observed for compound (I-1) at all tested concentrations.

### Example 4: Effect of compound (I-1) on mitochondria biosynthesis

The effect of compound (I-1) on mitochondria biosynthesis was investigated in normal human dermal fibroblasts (NHDF). NHDF were obtained from PromoCell (Lot No. 006151; female Caucasian donor; age 61 years; from eyelid skin) and cultivated in Fibroblast Growth Medium (PromoCell; Cat. No. C-023020) supplemented with Supplement Mix (PromoCell; Cat. No. C-039215) at 37 °C / 5 % CO₂. NHDF were seeded in flat-bottom 96-well plates (5000 cells / well) and incubated in said medium for 24 h or 48 h, respectively, with the compounds present in concentrations ranging from 0.03 µM to 200 µM. Mitochondria content was analyzed using the Mito-ID® Green Detection Kit for fluorescence microscopy (Enzo Life Sciences; Cat. No. ENZ-51055-K500). Fluorometric analysis was done using a Spectramax plate reader (Molecular Devices) and following the manufacturer's protocol. Cytotoxicity was determined in parallel by fluorometric readout using the Resazurin Based *In Vitro* Toxicology Assay Kit (Sigma Aldrich, Cat. No. TOX-8), and following the manufacturer's protocol.

### Results:

The total content of mitochondria in NHDF was significantly enhanced by compound (I-1) after incubation for 24 h and 48 h, respectively, while no cytotoxicity or loss of cell viability was observed. Significant enhancement of mitochondria biosynthesis was observed at concentrations of about 10 µM or higher.

**Table 9: Mitochondria content of NHDF after cultivation with compound (I-1)**

| | 24 h incubation time | | 48 h incubation time | |
|---|---|---|---|---|
| Conc. [µM] | Signal intensity [% Control] | Standard deviation | Signal intensity [% Control] | Standard deviation |
| 0.03 | 100 | 1.8 | 97 | 5.9 |
| 0.09 | 99 | 1.3 | 92 | 0.4 |
| 0.27 | 100 | 2.3 | 91 | 1.2 |
| 0.82 | 101 | 1.5 | 93 | 1.3 |
| 2.47 | 103 | 3.1 | 94 | 1.3 |
| 7.41 | 114 | 3.8 | 103 | 2.3 |
| 22.22 | 182 | 21.7 | 147 | 7.1 |
| 66.67 | 354 | 62.0 | 293 | 23.5 |
| 200.00 | 906 | 64.4 | 765 | 62.5 |

## Claims

1. Use of a compound of general formula (I) for cosmetic skin care 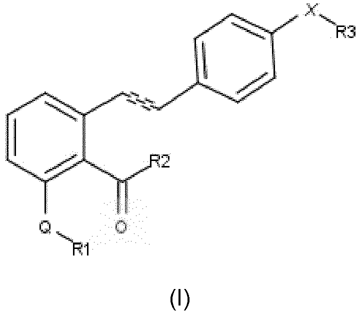 wherein 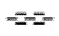 is a single or double bond;
R¹ is independently hydrogen or a linear, branched or cyclic, saturated or unsaturated C₁₋₆-aliphatic residue, optionally substituted with at least one substituent selected from the group consisting of oxo (=O), -OH, -O-C₁₋₆-alkyl, -C₁₋₆-alkyl, -O-benzyl, phenyl, F, Cl, and Br;
R² is independently hydrogen or a linear, branched or cyclic, saturated or unsaturated C₁₋₆-aliphatic residue, optionally substituted with at least one substituent selected from the group consisting of oxo (=O), -OH, -O-C₁₋₆-alkyl, -C₁₋₆-alkyl, -O-benzyl, phenyl, F, Cl, and Br or an aromatic or heteroaromatic 6-membered ring, optionally substituted with at least one substituent selected from the group consisting of-OH, -O-C₁₋₆-alkyl, -C₁₋₆-alkyl, -O-benzyl, phenyl, F, Cl, and Br:
or is A-R⁴ wherein
R⁴ is independently hydrogen or a linear, branched or cyclic, saturated or unsaturated C₁₋₆-aliphatic residue, optionally substituted with at least one substituent selected from the group consisting of oxo (=O), -OH,-O-C₁₋₆-alkyl, -C₁₋₆-alkyl, -O-benzyl, phenyl, F, Cl, and Br;
A is O or NR⁵ wherein
R⁵ is independently hydrogen or a linear, branched or cyclic, saturated or unsaturated C₁₋₆-aliphatic residue, optionally substituted with at least one substituent selected from the group consisting of oxo (=O), -OH, -O-C₁₋₆-alkyl, -C₁₋₆-alkyl, -O-benzyl, phenyl, F, Cl, and Br;
R³ is independently hydrogen or a linear, branched or cyclic, saturated or unsaturated C₁₋₆-aliphatic residue, optionally substituted with at least one substituent selected from the group consisting of oxo (=O), -OH, -O-C₁₋₆-alkyl, -C₁₋₆-alkyl, -O-benzyl, phenyl, F, Cl, and Br, or an aromatic or heteroaromatic 6-membered ring, optionally substituted with at least one substituent selected from the group consisting of-OH, -O-C₁₋₆-alkyl, -C₁₋₆-alkyl, -O-benzyl, phenyl, F, Cl, and Br;
X is O or NR⁶ wherein
R⁶ is independently as defined above for R⁵;
Q is O or NR⁷ wherein
R⁷ is independently as defined above for R⁵; and
wherein each of the phenyl rings in the formula (I) can have one or more further substituents selected from OH, NH₂, NHR⁸ wherein R⁸ is independently as defined above for R⁵, NR⁹R¹⁰ wherein R⁹,R¹⁰ are independently as defined above for R⁵; -O-C₁₋₆-alkyl, -C₁₋₆-alkyl, -O-C₂₋₆-alkenyl, C₂₋₆-alkenyl, C₄₋₆-cycloalkyl, 4- to 6-membered heterocycloalkyl, benzyl, -O-benzyl, phenyl, 4 to 6-membered heteroaryl, F, Cl, Br,-CN, -SO₃H, -COOH and corresponding C₁₋₆-alkyl esters and C₁₋₆-alkyl amides, sulfonamides, and O- or-C-glycosidic bound sugars;
optionally, in the form of the corresponding cosmetically and/or dermatologically acceptable salts and/or solvates thereof.

2. The use of claim 1 wherein
R¹ is hydrogen or C₁₋₃-alkyl;
R² is C₁₋₆-alkyl or -O-C₁₋₆-alkyl;
X is O;
Q is O; and
R³ is C₁₋₆-alkyl.

3. The use of claim 1 or 2 wherein 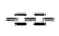 is a trans-configurated double bond.

4. The use according to any one of the preceding claims wherein the compound has the following structure:

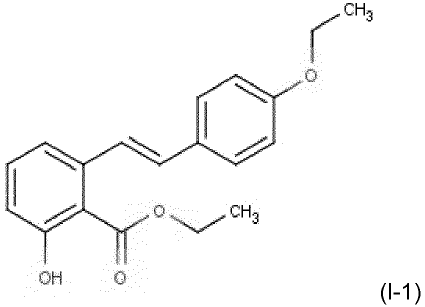

5. A compound as defined in any one of the preceding claims for use in the treatment of diabetic skin.

6. Use of the compound as defined in any one of claims 1 to 4 for activating sirtuins and/or enhancing AMPK phosphorylation and/or increasing the number of mitochondria in cells, preferably skin cells, *in vitro.*

7. A method for cosmetic skin care comprising the step of topically applying an effective amount of a cosmetic composition comprising a compound as defined in any one of claims 1 to 4 together with at least one cosmetically or dermatologically acceptable carrier, excipient and/or diluent onto human skin.

8. The method of claim 7 for the prevention and/or treatment of skin aging.

## Patentansprüche

1. Verwendung einer Verbindung der allgemeinen Formel (I) zur kosmetischen Hautpflege 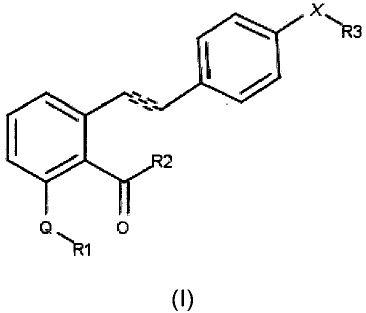 wobei 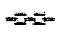 eine Einfach- oder Doppelbindung ist;
R¹ unabhängig Wasserstoff oder ein geradkettiger, verzweigter oder cyclischer, gesättigter oder ungesättigter C₁₋₆-aliphatischer Rest ist, der ggf. mit mindestens einem Substituenten substituiert ist, der aus der Gruppe, bestehend aus oxo (=O), -OH, -O-C₁₋₆-Alkyl, -C₁₋₆-Alkyl, -O-Benzyl, Phenyl, F, Cl und Br, ausgewählt ist;
R² unabhängig Wasserstoff oder ein geradkettiger, verzweigter oder cyclischer, gesättigter oder ungesättigter C₁₋₆-aliphatischer Rest, der ggf. mit mindestens einem Substituenten substituiert ist, der aus der Gruppe, bestehend aus oxo (=O), -OH, -O-C₁₋₆-Alkyl, -C₁₋₆-Alkyl, -O-Benzyl, Phenyl, F, Cl und Br, ausgewählt ist, oder ein aromatische oder heteroaromatischer 6-gliedriger Ring, der ggf. mit mindestens einem Substituenten substituiert ist, der aus der Gruppe, bestehend aus -OH, -O-C₁₋₆-Alkyl, -C₁₋₆-Alkyl,-O-Benzyl, Phenyl, F, Cl und Br, ausgewählt ist,
oder A-R⁴ ist, wobei
R⁴ unabhängig ein geradkettiger, verzweigter oder cyclischer, gesättigter oder ungesättigter C₁₋₆-aliphatischer Rest, der ggf. mit mindestens einem Substituenten substituiert ist, der aus der Gruppe, bestehend aus oxo (=O), -OH, -O-C₁₋₆-Alkyl, -C₁₋₆-Alkyl, -O-Benzyl, Phenyl, F, Cl und Br, ausgewählt ist;
A O oder NR⁵ ist, wobei
R⁵ unabhängig Wasserstoff oder ein geradkettiger, verzweigter oder cyclischer, gesättigter oder ungesättigter C₁₋₆-aliphatischer Rest ist, der ggf. mit mindestens einem Substituenten substituiert ist, der aus der Gruppe, bestehend aus oxo (=O), -OH, -O-C₁₋₆-Alkyl, -C₁₋₆-Alkyl, -O-Benzyl, Phenyl, F, Cl und Br, ausgewählt ist;
R³ unabhängig Wasserstoff oder ein geradkettiger, verzweigter oder cyclischer, gesättigter oder ungesättigter C₁₋₆-aliphatischer Rest, der ggf. mit mindestens einem Substituenten substituiert ist, der aus der Gruppe, bestehend aus oxo (=O), -OH, -O-C₁₋₆-Alkyl, -C₁₋₆-Alkyl, -O-Benzyl, Phenyl, F, Cl und Br, ausgewählt ist, oder ein aromatischer oder heteroaromatischer 6-gliedriger Ring ist, der ggf. mit mindestens einem Substituenten substituiert ist, der aus der Gruppe, bestehend aus -OH, -O-C₁₋₆-Alkyl, -C₁₋₆-Alkyl,-O-Benzyl, Phenyl, F, Cl und Br, ausgewählt ist,
X O oder NR⁶ ist, wobei
R⁶ unabhängig wie zuvor für R⁵ definiert ist;
Q O oder NR⁷ ist, wobei
R⁷ unabhängig wie zuvor für R⁵ definiert ist; und
wobei jeder der Phenylringe in der Formel (I) einen oder mehrere weitere Substituenten aufweisen kann, der/die aus der Gruppe, bestehend aus OH, NH₂, NHR⁸, wobei R⁸ unabhängig wie zuvor für R⁵ definiert ist, NR⁹R¹⁰, wobei R⁹, R¹⁰ unabhängig wie zuvor für R⁵ definiert sind; -O-C₁₋₆-Alkyl, -C₁₋₆-Alkyl, -O-C₂₋₆-Alkenyl, -C₂₋₆-Alkenyl, -C₄₋₆-Cycloalkyl, 4- bis 6-gliedriges Heterocycloalkyl, Benzyl, -O-Benzyl, Phenyl, 4- bis 6-gliediges Heteroaryl, F, Cl, Br, -CN, -SO₃H, -COOH und entsprechende C₁₋₆-Alkylester und C₁₋₆-Alkylamide, Sulfonamide und O- oder C-glykosidische Zucker, ausgewählt ist/sind;
ggf. in Form von deren entsprechenden kosmetisch und/oder dermatologisch verträglichen Salzen und/oder Solvaten.

2. Verwendung nach Anspruch 1, wobei
R¹ Wasserstoff oder -C₁₋₃-Alkyl ist;
R² -C₁₋₆-Alkyl oder -O-C₁₋₆-Alkyl ist;
X O ist:
Q O ist; und
R³ C₁₋₆-Alkyl ist.

3. Verwendung nach Anspruch 1 oder 2, wobei 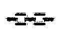 eine trans-konfigurierte Doppelbindung ist.

4. Verwendung nach einem der vorhergehenden Ansprüche, wobei die Verbindung die folgende Struktur aufweist:

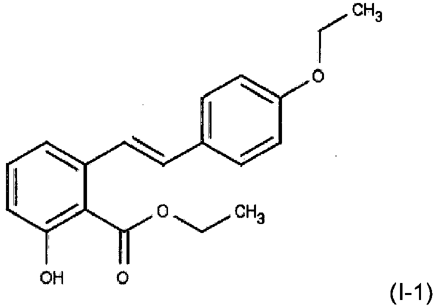

5. Verbindung gemäß der Definition in einem der vorhergehenden Ansprüche zur Verwendung in der Behandlung diabetischer Haut.

6. Verwendung der Verbindung gemäß der Definition in einem der Ansprüche 1 bis 4 zur Aktivierung von Sirtuinen und/oder Verstärkung der AMPK-Phosphorylierung und/oder Ehöhung der Anzahl von Mitochondrien in Zellen, vorzugsweise Hautzellen, *in vitro.*

7. Verfahren zur kosmetischen Hautpflege, das den Schritt der topischen Anwendung einer wirksamen Menge einer kosmetischen Zusammensetzung, umfassend eine Verbindung gemäß der Definition in einem der Ansprüche 1 bis 4 zusammen mit mindestens einem kosmetisch und/der dermatologisch verträglichen Träger, Hilfsstoff und/oder Verdünnungsmittel, auf die menschliche Haut umfasst.

8. Verfahren nach Anspruch 7 zur Prävention und/oder Behandlung der Hautalterung.

## Revendications

1. Utilisation d'un composé de formule générale (I) pour des soins cosmétiques de la peau 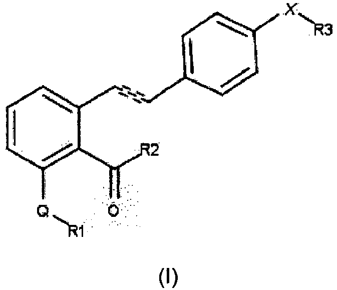 où 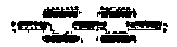
est une liaison simple ou double ;
R¹ représente indépendamment un atome d'hydrogène ou un résidu aliphatique en C₁₋₆ linéaire, ramifié ou cyclique, saturé ou insaturé, facultativement substitué par au moins un substituant choisi dans le groupe comprenant oxo (=O), -OH, -O-alkyle en C₁₋₆, -alkyle en C₁₋₆, -O-benzyle, phényle, F, Cl et Br ;
R² représente indépendamment un atome d'hydrogène ou un résidu aliphatique en C₁₋₆ linéaire, ramifié ou cyclique, saturé ou insaturé, facultativement substitué par au moins un substituant choisi dans le groupe comprenant oxo (=O), -OH, -O-alkyle en C₁₋₆, -alkyle en C₁₋₆, -O-benzyle, phényle, F, Cl et Br ou un cycle à 6 chaînons aromatique ou hétéroaromatique, facultativement substitué par au moins un substituant choisi dans le groupe comprenant -OH, -O-alkyle en C₁₋₆, -alkyle en C₁₋₆, -O-benzyle, phényle, F, Cl et Br :
ou est A-R4, où
R⁴ représente indépendamment un atome d'hydrogène ou un résidu aliphatique en C₁₋₆ linéaire, ramifié ou cyclique, saturé ou insaturé, facultativement substitué par au moins un substituant choisi dans le groupe comprenant oxo (=O), -OH, -O-alkyle en C₁₋₆, -alkyle en C₁₋₆, -O-benzyle, phényle, F, Cl et Br ;
A est O ou NR⁵ où
R⁵ représente indépendamment un atome d'hydrogène ou un résidu aliphatique en C₁₋₆ linéaire, ramifié ou cyclique, saturé ou insaturé, facultativement substitué par au moins un substituant choisi dans le groupe comprenant oxo (=O), -OH, -O-alkyle en C₁₋₆, -alkyle en C₁₋₆, -O-benzyle, phényle, F, Cl et Br ;
R³ est indépendamment un atome d'hydrogène ou un résidu aliphatique en C₁₋₆ linéaire, ramifié ou cyclique, saturé ou insaturé, éventuellement substitué par au moins un substituant choisi dans le groupe comprenant oxo (=O), -OH, -O-alkyle en C₁₋₆, -alkyle en C₁₋₆, -O-benzyle, phényle, F, Cl et Br, ou un cycle à 6 chaînons aromatique ou hétéroaromatique, facultativement substitué par au moins un substituant choisi dans le groupe comprenant -OH, -O-alkyle en C₁₋₆, -alkyle en C₁₋₆, -O-benzyle, phényle, F, Cl et Br ;
X est O ou NR⁶ où
R⁶ est indépendamment tel que défini ci-dessus pour R⁵ ;
Q est O ou NR⁷ où
R⁷ est indépendamment tel que défini ci-dessus pour R⁵ ; et
dans lequel chacun des cycles phényle dans la formule (I) peut avoir un ou plusieurs substituants supplémentaires choisis parmi OH, NH₂, NHR⁸, où R⁸ est indépendamment tel que défini ci-dessus pour R⁵, NR⁹R¹⁰, où R⁹, R¹⁰ sont indépendamment tels que définis ci-dessus pour R⁵ ; -O-alkyle en C₁₋₆,-alkyle en C₁₋₆, -O-alcényle en C₂₋₆, alcényle en C₂₋₆, cycloalkyle en C₄₋₆, hétérocycloalkyle de 4 à 6 chaînons, benzyle, -O-benzyle, phényle, hétéroaryle de 4 à 6 chaînons, F, Cl, Br, -CN, -SO₃H, -COOH et des esters d'alkyle en C₁₋₆ correspondants et des alkylamides en C₁₋₆, des sulfonamides, et des sucres liés O- ou C-glycosidiques ;
facultativement, sous la forme de sels et/ou solvates correspondants de ceux-ci cosmétiquement et/ou dermatologiquement acceptables.

2. Utilisation selon la revendication 1, dans laquelle
R¹ est un atome d'hydrogène ou un groupe alkyle en C₁₋₃ ;
R² est un alkyle en C₁₋₆ ou -O-alkyle en C₁₋₆ ;
X est O ;
Q est O ; et
R³ est un alkyle en C₁₋₆.

3. Utilisation selon la revendication 1 ou 2, dans laquelle 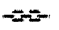 est une double liaison trans-configurée.

4. Utilisation selon l'une quelconque des revendications précédentes, dans laquelle le composé a la structure suivante :

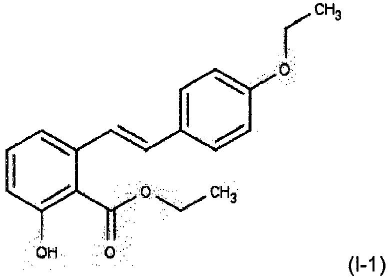

5. Composé tel que défini dans l'une quelconque des revendications précédentes, destiné à être utilisé dans le traitement de la peau diabétique.

6. Utilisation du composé tel que défini dans l'une quelconque des revendications 1 à 4, pour activer des sirtuines et/ou augmenter la phosphorylation de l'AMPK et/ou augmenter le nombre de mitochondries dans des cellules, de préférence des cellules de la peau, *in vitro.*

7. Procédé de soin cosmétique de la peau comprenant l'étape d'application topique d'une quantité efficace d'une composition cosmétique comprenant un composé tel que défini dans l'une quelconque des revendications 1 à 4, avec au moins un véhicule, excipient et/ou diluant acceptable du point de vue cosmétique ou dermatologique sur de la peau humaine.

8. Procédé selon la revendication 7 pour la prévention et/ou le traitement du vieillissement de la peau.
